Europäisches Patentamt

European Patent Office

Office européen des brevets

(11) Veröffentlichungsnummer: **0 193 769**
A1

## (12) EUROPÄISCHE PATENTANMELDUNG

(21) Anmeldenummer: 86101965.1

(22) Anmeldetag: 17.02.86

(51) Int. Cl.4: **C12N 13/00** , C12N 5/00 , C12N 15/00 , //A61K39/395

(30) Priorität: 02.03.85 DE 3507398

(43) Veröffentlichungstag der Anmeldung:
10.09.86 Patentblatt 86/37

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Trawinski, Jürgen, Dr.
4230 San Pablo Dam Road
El Sobrante, CA 94803(US)
Erfinder: Hübner, Günter, Dr.
Ursulastrasse 7
D-5600 Wuppertal 1(DE)
Erfinder: Paffhausen, Wolfgang, Dr.
Dürscheider Weg 17
D-5090 Leverkusen 3(DE)
Erfinder: Opitz, Hans-Georg, Dr.
140 Clover Hill Court
Danville, CA 94526(US)
Erfinder: Opitz, Uta, Dr.
140 Clover Hill Court
Danville, CA 94526(US)
Erfinder: Zembrod, Alfred, Dr.
Nittumer Weg 60
D-5060 Bergisch-Gladbach 2(DE)
Erfinder: Schlumberger, Horst Dieter, Prof.Dr.
Schwartnerstrasse 5
D-5600 Wuppertal 2(DE)

(54) Methode zur Fusionierung biologischer Zellen mit Hilfe elektrischer Impulse.

(57) Es wird ein Verfahren zur elektrisch induzierten Zellfusion nach mechanisch erzeugtem Zellkontakt beschrieben sowie dessen Anwendung zur Herstellung von homologen und heterologen Hybridomzellen, die Antikörper produzieren.

EP 0 193 769 A1

## Methode zur Fusionierung biologischer Zellen mit Hilfe elektrischer Impulse

Die vorliegende Erfindung betrifft ein Verfahren zur elektrischinduzierten Zellfusion nach mechanisch erzeugtem Zellkontakt, sowie dessen Anwendung zur Herstellung monoklonaler Antikörper.

Durch die Fusion von Zellen kann eine Neukombination von Eigenschaften erreicht werden. Dieses hat in den letzten Jahren z.B. zu der Entwicklung von monoklonalen Antikörpern geführt, die in der Medizin und in der biologischen Grundlagenforschung viele neue Möglichkeiten eröffnen.

Mit den chemischen Methoden der Zellfusion mittels Polyethylenglykol oder geeigneter Viren können eine Vielzahl von verschiedenen Zelltypen miteinander verschmolzen werden, jedoch sind bei vielen Zellen die Fusionsfrequenzen relativ niedrig.

Eine universelle Anwendbarkeit ist durch eine rein physikalische Fusionsmethode gegeben. Hierbei werden durch kurze elektrische Pulse hoher Amplitude Zellmembranen zum reversiblen Zusammenbruch gebracht. An solchen Stellen mit gestörter Membranstruktur kann es bei vorhandenem Membrankontakt zur Zellfusion kommen.

Der für die Durchführung der Electrofusion erforderliche Zellkontakt kann auf verschiedenen Wegen hergestellt werden. In dem von U. Zimmermann publizierten Elektrofusionssystem (Zimmermann, U. 1982, Biochim., Biophys., Acta 694, 227 -227) wird der Zellkontakt mit Hilfe inhomogener elektrischer Wechselfelder erreicht - (Dielektrophorese). Die Zellfusion wird dann durch die Applikation eines kurzen elektrischen Pulses hoher Amplitude erreicht.

Neumann erhielt Zellfusionen nach Elektropulsapplikation auf Suspensionen von Dictyostelium Zellen (Neumann, E., Gerisch, G. and Opatz, K. 1980, Naturwissenschaften 67, 414 -415). Hierbei handelt es sich um selbstaggregierende Zellen, die durch diese Eigenschaft in Suspension bereits den für die Zellfusion erforderlichen Zellkontakt besitzen.

In anderen Versuchen wurde von Teissié gezeigt, daß durch Pulsapplikation auf Monolayerkulturen von 3T3-Fibroblasten in dem Zellrasen Zellfusionen, erzeugt werden konnten (Teissié, J. et al, 1982, Science, Vol. 216, 537 -538). In diesem Fall liegt bereits wegen des Wachstumsverhaltens der Zellen (Monolayer) ein enger Membrankontakt in der Kultur vor, so daß keine besonderen Maßnahmen erforderlich sind um die Zellen in Kontakt zu bringen.

Eine weitere Methode zur Herstellung von Zellkontakten in Zellsuspensionen wurde von Lo et al. 1984 beschrieben (Lo, M.M.S. et al, 1984 Nature, Vol. 310, 792 - 794). Dazu wird ein künstlich hergestellter Avidin-Antigen-Komplex mit Milzzellen gemischt, wobei sich das Antigen an die membranständigen antigenspezifischen Antikörper der Lymphocyten anlagert. Das am Antigen hängende Avidin bindet an Biotin, welches an die Membran von Myelomzellen gekoppelt wurde. Damit wird ein spezifischer Kontakt zwischen Myelomzellen und Lymphocyten hergestellt.

Wie oben schon erwähnt, ist eine Methode um eine größere Anzahl von Zellkontakten zu erhalten, die Dielektrophorese. Hierbei werden die zu fusionierenden Zellen mit Hilfe inhomogener Wechselfelder angesammelt und so in engen Membrankontakt gebracht.

Um aber ein solches Ansammeln der Zellen zu erreichen, muß die Wärmeentwicklung, ausgelöst durch den Stromfluß, minimiert werden, denn durch thermische Bewegung und die damit verbundenen Turbulenzen würde der Zellkontakt gestört. Aus diesem Grunde arbeitet U. Zimmermann in einem ionenarmen Medium, wie z.B. isotonische Mannitlösung (Zimmermann, U., 1982, Biochim., Biophys., Acta 694, 227 -227; Zimmermann, U., et al., 1981, Angew. Chemie 93, 332 -351).

Dabei müssen die Zellen vor der Fusion in ein derartiges Medium überführt werden. Die Verwendung solcher Lösungen hat aber Zellschädigungen zur Folge. Darüber hinaus erlaubt die für Dielektrophorese und die Pulsapplikation verwendete Fusionskammer wegen ihrer Größe und ihrer Konstruktion nur die Fusion weniger Tausend Zellen. Eine Herstellung antikörperproduzierender Hybridome konnte mit dieser Methode bisher nicht gezeigt werden.

Nachfolgend wird eine Methode zur Herstellung von Hybridomzellen mittels Elektrofusion beschrieben, deren Besonderheit darin liegt, daß der für die Elektrofusion notwendige Zellkontakt durch ein mechanisches Verfahren herbeigeführt wird.

Solche Verfahren können sein: Filtration, Dialyse oder Sedimentation. Besonders bevorzugt ist dabei die Herstellung von Zellkontakten durch Zentrifugation. Dadurch wird ein dichtgepacktes Zellsediment gewonnen.

Anschließend wird die Zellfusion durch die Applikation elektrischer Impulse induziert.

Aus der Literatur ist bekannt, daß für die Elektrofusion zwischen zwei Zellen zu Beginn des Elektroimpulses mindestens lokal ein homogenes elektrisches Feld existieren muß (Zimmermann, U., 1982, Biochim., Biophys., Acta 694, 227 -277). Da die Zellmembran als elektrischer Isolator, Innen- und Außenraum von Zellen aber als elektrisch leitend betrachtet werden können, kommt es im Verlaufe der zeitlichen Entwicklung des Elektroimpulses zu einer starken Polarisierung der Zellmembranen, als deren Folge ein elektrischer Durchbruch stattfindet. Die Polarisation bedingt lokale Feldverzerrungen eines sonst homogenen elektrischen Feldes. In einem dichten Sediment von biologischen Zellen muß man aber mit starken Feldverzerrungen und Inhomogenitäten rechnen, die die elektrische Feldkonfiguration äußerst unübersichtlich machen. Unter diesen Umständen war es überraschend, daß das Verfahren der Elekrofusion auf dichte Zellsediemente erfolgreich angewendet werden kann.

Das gesamte erfindungsgemäße Verfahren kann in normalem Zellkulturmedium durchgeführt werden, da die Dielektrophorese und damit der Mediumwechsel entfällt. Eine Schädigung der Zellen durch unphysiologische Medien wie z.B. Mannitlösung oder durch die Wechselfelder der Dielektrophorese ist demnach ausgeschlossen.

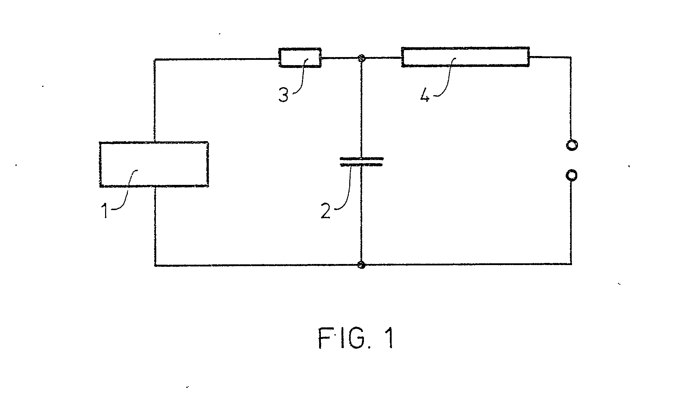

Der bei den Versuchen benutzte und in Fig. 1 beschriebene elektrische Impulsgenerator zur Erzeugung kurzer elektrischer Hochspannungsimpulse besteht aus einem Hochspannungsgerät (1), mit dem ein Kondensator (2) über einen Widerstand (3) aufgeladen wird, sowie einem - schnellen Hochspannungsschalter in Form einer Wasserstoff-gefüllten Thyratron-Röhre (4), die durch mauelle Bedienung geschaltet werden kann.

Die erzeugten Impulse haben je nach Kondensator-Kapazität und Widerstand der biologischen Suspension eine Halbwertsbreite zwischen 5 μs und 100 μs sowie eine Impulshöhe bis zu 3 kV. Die Elektrodenanordnung bestand aus zwei, im Abstand von 2 mm angeordneten parallelen Platinblechen von 2 x 5 mm Größe. Die beschriebene Methode wurde von uns zur Herstellung monoklonaler Antikörper-produzierender Hybridomzellen eingesetzt.

Dazu werden Myelomzellen und Lymphocyten miteinander fusioniert. Die beiden Zelltypen werden im Zellkulturmedium gemischt, wobei sich Mischungsverhältnisse von 1:10 bis 10:1 als geeignet erwiesen haben.

Als Medium kann jedes für die verwendeten Zellen geeignetes Kulturmedium verwendet werden. ·

Der Zusatz von Proteinen wie Rinderserumalbumin - (BSA), Ovalbumin u.a. zum Fusionsmedium beeinflußt die Ausbeute an fusionierten Zellen. Der Zusatz von Proteinen zum Medium erhöht die Fusionsausbeute im Vergleich zu proteinfreiem Medium. Nach Überschreiten einer optimalen Proteinkonzentration sinkt die Fusionsausbeute wieder ab.

Geeignet erwiesen sich Zusätze von 0,02 % -2 % BSA zum Fusionsmedium. Bevorzugt wurde bei 0,1 % BSA gearbeitet. Der Zusatz von FCS (foetales Kälberserum) wirkt sich ebenso auf die Fusionsausbeute aus. Bei Zusatz von 20 -40 % FCS wird die Fusionsausbeute im Vergleich zu höheren oder geringeren Konzentrationen bei gleichen Pulsparametern erhöht. Der Bereich von 0 % bis 100 % FCS kann für die Fusion verwendet werden. Besonders geeignet erweisen sich Konzentrationen von 0 % -50 % FCS. Bevorzugt wurde bei Zusatz von 10 - 30 % FCS gearbeitet.

Die in diesem Medium gemischten Zellen wurden bei hohen g-Zahlen in kurzer Zeit abzentrifugiert. Für die Zentrifugation wurden 1,5 ml Eppendorfgefäße verwendet. g-Zahlen von 200 xg bis 2200 xg erwiesen sich bei Zentrifugationszeiten von 10 Minuten bis 5 Sekunden als geeignet. Bevorzugt wurden die Zellen für 8 Sekunden bei 2000 xg in einer Eppendorf-Zentrifuge sedimentiert. Anschließend wurden die Elektroden in das Sediment getaucht und die Fusion durch die Applikation der Elektroimpulse herbeigeführt. 60 Sekunden nach der Pulsapplikation werden die Elektroden vorsichtig aus dem Zellsediment entfernt. Eine frühere Entnahme ist nicht empfehlenswert, da die Zellkontakte durch entstehende Aufwirbelung vor Abschluß des Fusionsprozesses zerstört würden.

Nach der Elektrodenentnahme wird vorsichtig Zellkulturmedium auf das Zellpellet geschichtet, ohne daß die Zellen aufgewirbelt werden. Die Zellen werden danach noch 1 -60 Minuten bei etwa 15°C bis 38°C inkubiert, bevor sie resuspendiert und in geeignete Kulturgefäße überführt werden. Die besten Ergebnisse ergaben sich bei Inkubationszeiten von 20 -40 Minuten bei Raumtemperatur (RT).

Das Wachstum von Hybridklonen ist in einem nach dem Stand der Technik bekannten und geeigneten Selektionsmedium nach einigen Tagen zu beobachten.

Vorteile des erfindungsgemäßen Verfahrens gegenüber anderen Methoden zur Herstellung von Zellkontakten für Elektrofusion:

Von keinem bisher beschriebenen Verfahren zur Erzeugung von Hybridomen mittels Elektrofusion ist bislang bekannt, daß die fusionierten Zellen Antikörper produziert haben. Eine Ausnahme bildet die Arbeit von Lo et al (Lo M.M.S. et al. 1984 Nature, Vol. 310, 792 -794). Das erfindungsgemäße Verfahren hat nachweislich zur Antikörper-Produktion geführt, wobei die Antikörper-Produktionsrate wesentlich höher lag als bei den von Lo et al. berichteten Versuchen. Ferner ist das erfindungsgemäße Verfahren im Vergleich zu Lo wesentlich einfacher und ohne chemische Manipulationen der Zellen durchführbar. Gegenüber den anderen Verfahren der Zellfusion wie z.B. mit Hilfe von Polyethylenglykol (PEG) oder mit Hilfe von Viren ist ebenfalls keine chemische Beeinflussung der Zellen erforderlich, die Zellschäden hervorrufen kann. Außerdem ist die Gefahr einer Virusfreisetzung nicht gegeben.

Mit Hilfe des erfindungsgemäßen Verfahrens werden zudem höhere Fusionsfrequenzen vitaler Hybride als mit allen anderen Methoden erreicht. Bei der vorgestellten Methode handelt es sich um eine Universalmethode für Fusionsprozesse unabhängig vom Zelltyp. Sie ist eine Bereicherung bekannter Methoden zur Herstellung monoklonaler Antikörper durch eine einfachere und effizientere Methode.

1. Beispiel

Maus/Maus Hybridome

Fusioniert wurde die HGPRT-negative Maus Myelomzellinie X63 Ag 8653 mit Maus-Milzzellen.

Balb/c Mäuse wurden durch wiederholte i.p. Injektionen von Flußneunaugen-Dotterprotein (Lampetra fluviatilis Vitellin) gegen dieses Protein immunisiert.

Je Injektion bekamen sie 300 μg Lampetra fluviatilis Vitellin in Phosphat gepufferte Kochsalzlösung (PBS). 6 Tage nach der ersten Injektion erhielten die Tiere weitere 100 μg Vitellin i.p. appliziert. Eine Kontrolle der Immunantwort erfolgte 3 Tage nach dieser 2. Injektion durch Bestimmung von antigenspezifischen Serum-Antikörpern im Radioimunassay (RIA). Das erforderliche Blut wurde durch Punktion des retroorbitalen Venenplexus gewonnen. Eine dritte Injektion mit 100 μg Vitellin erfolgte nach weiteren 10 Tagen. 3 -6 Tage nach der 3. Antigeninjektion wurden die Tiere in gesättigter $CO_2$-Atmosphäre abgetötet und die Milz steril entnommen und durch Zerreiben in Einzelzellsuspensionen zerlegt. Die Zellen wurden 2 mal mit BSS (balanced salt solution) gewaschen, anschließend in dem Kulturmedium RPMI 1640® (KC-Biological) Medium/10 % FCS aufgenommen und mit X63-Zellen gemischt. Alle verwendeten Zellen wurden in RPMI 1640® Medium (KC Biological) mit 10 % RCS, 30 iE/ml Penicillin, 30 μg/ml Streptomycin - (KC Biological) und 2 mM Glutamin (KC Biological) bei 37°C und 5 % $CO_2$ kultiviert.

Besonders hohe Fusionsausbeuten ergaben sich bei 2- bis 20-fachem Lymphocytenüberschuß gegenüber den Myelomzellen. Als ganz besonders geeignet erwiesen sich Mischungsverhältnisse von 1 Myelomzelle:2 Lymphocyten bis 1Myelomzelle:8 Lymphocyten.

Im nachfolgend beschriebenen Versuchsansatz wurden 1.5 x $10^7$ X63-Zellen und 3.0 x $10^7$ Milzzellen gemischt und in einem Reaktionsgefäß nach Eppendorf für 8" in einer Eppendorf-Zentrifuge bei 2000 x g zentrifugiert. Der Überstand wurde restlos entfernt und die in Abb. 1 dargestellte Elektrodenanordnung in das Zellpellet gesteckt.

In den 4 dargestellten Versuchen wurden jeweils 2 Pulse im Abstand von 60 Sekunden auf die Zellen appliziert. Der Kontrollansatz wurde wie die Versuchsansätze behandelt, erhielt jedoch keine Pulse appliziert.

30" nach dem letzten Puls wurden die Elektroden vorsichtig aus dem Zellsediment entfernt und die Zellen mit 1 ml RPMI 1640® +10 % FCS überschichtet. Nach einer 60-minütigen Inkubationszeit bei Raumtemperatur wurde das Sediment vorsichtig resuspendiert und die Zellen mit einer Endkonzentration von 1 x $10^6$ Zellen/ml verdünnt und auf Kulturplatten (Costar) verteilt.

Die Selektion von X63-Hybridomzellen erfolgte im HAT-Selektionsmedium. Dem normalen RPMI 1640® Medium (10 % FCS) wurde dazu 5 μM Aminopterin, 100 μM Hypoxanthin und 40 μM Thymidin (alle drei von Flow Lab.) zugesetzt.

Tabelle 1:

| Fus.-Nr. | Ampl. kV/cm | Pulszahl | Anzahl Klone | Ig. pos. (%) | Antigen pos. (%) |
|---|---|---|---|---|---|
| 45 | 0 | 0 | 0 | 0 | 0 (Kontrollansatz) |
| | 2,5 | 2 | 233 | 58 | 29 |
| | 3,0 | 2 | 183 | 42 | 25 |
| | 3,5 | 2 | 22 | nd | nd |
| | 4,0 | 2 | 24 | nd | nd |

Die Halbwertsbreite der Pulse betrug in allen Fällen 50 μsek.

Die Kulturüberstände der entstehenden Klone wurden nach erfolgter Reklonierung in einem RIA auf Immunglobulinen überprüft.

Die Hybridklone, die Antikörper in das Medium sezernierten, wurden in einem spezifischen Radio-Immunassay - (RIA) auf Antigenspezifität überprüft.

Der RIA wurde in Einweggefäßen (FLOW M 17451) nach folgendem Schema durchgeführt:

-Beschichten des Rundbodens der Gefäße mit 100 μl Lampetra fluvialitis Vitellin (1 mg/ml in PBS) bei 4°C, 24 Stunden.

-Dreimaliges Waschen der Gefäße mit phosphatgepufferter Kochsalz (PBS) + 3 % BSA

-Inkubation für 2 Stunden bei Raumtemperatur mit PBS, 3 % BSA

-Dreimaliges Waschen mit PBS, 0,3 % BSA

-Zugabe von 100 μl des zu testenden Kulturüberstandes für 2 Stunden bei Raumtemperatur

-Dreimaliges Waschen der Gefäße mit PBS (0,3 % BSA)

-Zugabe von 100 μl $^{125}$J-markiertem anti-Mausimmunglobu

lin (entspr. 120 000 cpm, goat anti mouse Ig, 100 μ Ci/ml NEX 159, NEN) für 2 Stunden bei Raumtemperatur

-Fünfmaliges Waschen der Gefäße mit PBS

-Messen der Aktivität in einen γ -Zähler (LKB 1270)

Von den antigenspezifisch produzierenden Klonen wurden die 22 weiter kultiviert, die die meisten Antikörper ins Medium sezernierten. Nach 10 in vitro Passagen wurden diese Zellen i.p. in Balb/c Mäuse injiziert, um Ascites zu erzeugen. Nach dieser Mauspassage hatten 13 der Klone die Immunglobulinproduktion eingestellt bzw. war der Antikörper vernachlässigbar gering.

Die verbliebenen 9 Klone wurden auf die Anzahl ihrer Chromosomen ebenso untersucht wie auf die Klasse der von ihnen produzierten anti-Dotterprotein-Antikörper.

Die Anzahl der Chromosomen liegt in allen Fällen über der, die für unfusionierte Zellen zu erwarten wäre. Die Bestimmung der Ig-Klassen erfolgte mit Hilfe eines Immunodiffusionstestes nach Ouchterlony unter Verwendung spezifischer Antiseren.

Wie Tab. 2 zeigt, produzieren zwei Klone Antikörper der Klasse IgM und sieben Klone produzieren IgGl Antikörper.

Tabelle 2:

| Klon-Nr. | Ig-Klasse | Chromosomen-zahl |
|---|---|---|
| 7 | G 1 | 79 |
| 8 | M | 78 |
| 9 | G 1 | 76 |
| 11 | G 1 | 88 |
| 18 | G 1 | 64 |
| 19 | M | 74 |
| 20 | G 1 | 76 |
| 21 | G 1 | nd |
| 22 | G 1 | 94 |
| X63 unfusioniert | | 46 |
| Mauslymph. | | 40 |

2. Beispiel:

Maus-Mensch Heterohybridome

Zur Herstellung von Heterohybridomen wurde die murine Myelomlinie X63 Ag 8653 mit menschlichen peripheren Blutlymphocyten (hPBL) fusioniert. Aufgrund der Enzymdefizienz der X63-Zellinie ist ein entstehendes Heterohybridom in HAT-Medium selektierbar.

Die hPBL wurden auf buffy coat (nach Zentrifugation von Vollblut den Erythrocyten aufliegenden Lymphocytenschicht) mit Hilfe einer Gradientenzentrifugation über Ficoll hypaque® gewonnen und nach Isolierung 2 mal mit BSS gewaschen.

Die Zellen wurden in RPMI 1640® Medium mit 10 % FCS aufgenommen und mit X63-Zellen im Verhältnis 2:1 gemischt mit einer Gesamtzellzahl von $1,5 \times 10^7$ Zellen/Versuch. Das Zellgemisch wurde für 10 Sekunden bei 2000 xg in einem Eppendorf Reaktionsgefäß in einer Eppendorf-Zentrifuge sedimentiert.

Im Anschluß an die Zentrifugation wurde das überstehende Medium volltändig entfernt und die in Abb. 1 dargestellte Elektrodenanordnung in das Zellsediment gesteckt.

3 Pulse von jeweils 3,5 kV/cm und 50 μsek Länge im Abstand von jeweils 60 Sekunden appliziert führten in dem Zellsediment zu Zellfusionen.

60 Sekunden nach der Applikation des letzten Pulses wurden die Elektroden vorsichtig aus dem Zellpellet entfernt und die Zellen mit RPMI 1640® Medium /10 % FCS überschichtet. Nach einer Inkubationszeit von 30 Minuten bei Raumtemperatur wurden die Zellen resuspendiert und in einer Endkonzentration von $1 \times 10^6$ Zellen/ml in RPMI + 10 % FCS + HAT in Costarplatten ausgesät.

Nach 11 Tagen wuchsen in den Kulturplatten die ersten Hybridklone aus. Nach 14 Tagen waren insgesamt 70 Klone im Selektionsmedium feststellbar.

Die morphologisch von den ebenfalls proliferierenden Lymphocyten gut unterscheidbaren Hybridzellen wuchsen bis auf eine Klonstärke von etwa 1000 -2000 Zellen heran. 20 -25 Tage nach der Fusion kam es bei den Zellen zu irregulär ablaufenden Zellteilungen und zum Absterben der Zellen.

3. Beispiel

Mensch-Maus Heterohybridome

Zur Testung auf Verwendbarkeit der enzymdefizienten (Hypoxanthinphosphoribosyltransferase neg.) humanen Myelomzellinie WI-L2c13 für Elektrofusion und Aufzucht von Hybridomen, wurden $10^7$ Myelomzellen mit $4 \times 10^7$ Milzzellen von Balb/c-Mäusen fusioniert. Dazu wurden die Zellen nach gemeinsamer Sedimentierung zweimal einem Elektro-Impuls von 4 kV/cm in einem 60 Sekunden Abstand ausgesetzt. 60 Sekunden nach dem zweiten Impuls wurden die Elektroden vorsichtig entfernt und die Zellen mit RPMI 1640® /10 % FCS überschichtet. Nach einer Stunde Inkubationszeit bei Raumtemperatur wurden die Zellen resuspendiert und in einer Konzentration von $10^5$ Zellen /0,2 ml in Mikrotiterplatten ausgesät, in die bereits einen Tag zuvor $10^5$ Maus-Milzzellen pro Mikrotitervertiefung als Feeder-Zellen eingegeben waren; als Selektionsmedium für die Anzucht von Hybridomen diente RPMI 1640® Medium mit 10 % FCS plus 50 μM Hypoxanthin und 10 μM Azaserin. Nach 13 Tagen wuchsen 4 Klone pro 10 Mikrotitervertiefung. Die Klone wurden nicht näher untersucht.

4. Beispiel:

Humane Hybridome

Jeweils $10^7$ WI-L2c13-Zellen in spät-logarithmischer Phase und unstimulierte humane mononukleare Zellen aus dem peripheren Blut (nach Ficoll-Trennung) wurden gemischt. Dem Zellpellet wurden 2 Pulse mit 5 kV/cm appliziert - (Zeitintervall 1 Min.) und dann 1 ml Kulturmedium (RPMI 1640® ÷ 10 % FCS) zugegeben. Nach 1 h erfolgte die Aussaat der Zellen in Mikrotiterplatten (Kulturvolumen 0.2 ml pro Vertiefung). Die Zusammensetzung des Aufzucht-Mediums war: RPMI 1640® supplementiert mit Penicillin - (100 U/ml), Streptomycin (100 µg/ml), L-Glutamin (2 mM), Hepes-Puffer (4 mM), 2-Mercaptoethanol (50 µM) und 10 % (v/v) fötales Kälberserum (FCS); zusätzlich Hypoxanthin (50 µM) und Azaserin (10 µM) (Buttin et al In: F. Melchers, M. Potter & N. Warner, Lymphocyte Hybridomas, Springer Verlag Berlin-Heidelberg-New York, 1979, 27 - 36). Inkubation der Kulturen erfolgte bei 37°C und 7.5 % $CO_2$ in feuchtigkeitsgesättigter Luft.

Nach 20 bis 26 Tagen wurden die Mikrotiterplatten auf Wachstum von Klonen hin untersucht und die Vertiefungen mit Klonwachstum auf Anwesenheit von Antikörpern im Kulturüberstand mit einem Sandwich-Elisa wie folgt geprüft. Anti-human IgG bzw. anti-human IgM Antikörper von der Ziege (Fa. Medac, Hamburg) wurden 1:100 mit PBS verdünnt und in Microelisa-Platten (Immunolon II) durch Inkubation über Nacht bei 4°C gekoppelt (100 µl/Vertiefung), und die Plastikoberfläche wurde mit 1 %

Ovalbuminlösung abgesättigt. Dann wurden 50 µl Zellkulturüberstände oder humanes Kontroll-IgG bzw. IgM - (Medac) oder Kulturmedium zur Inkubation über Nacht bei 4°C zugegeben.

Zum Nachweis spezifischer Bindung erfolgte die Beschichtung mit 50 µl anti-human IgG bzw. anti-hum IgM Ziegen-Antikörpern, an die Peroxidase konjugiert war (1:200 in PBS/Tween 20® [2000:1] verdünnt; Fa. Medac) und 2 h Inkubation bei 37°C.

Zwischen allen Schritten wurde jeweils 3 -5 mal mit PBS/Tween 20® gewaschen. Sodann wurden 50 µl einer $H_2O_2$/Chromogen-Lösung zugegeben (25 µl einer 0,1 % igen $H_2O_2$-Lösung in PBS pro 1 ml ABTS-Lösung [2,2'-Anzino-di-3-ethyl benzthiazoline-Sulfonsäure; 1 mg/ml Citratpuffer pH 4]).

Die Reaktion wurde mit 150 µl einer 0,5 %igen $NaN_3$-Lösung gestoppt und die Extinktion in einem Microelisa-Reader bei 405 nm gemessen.

Tab. 3 gibt a) die Anzahl der in einem Versuch erhaltenen Mikrotiter-Vertiefungen an, in denen Klone angewachsen waren, und b) den Anteil der Klone, bei denen im Überstand Antikörper nachweisbar waren.

Tabelle 3

Wachstum und Antikörper-Synthese humaner Hybridome

| Näpfe | Vertiefungen mit Klonen | IgM-pos. Überstände | IgG-pos. Überstände |
|---|---|---|---|
| 192 | 116 | 25 | 22 |

Die im Selektionsmedium aufgezogenen Zellen sind deutlich größer als die Zellen der Fusionspartner. Karyologische Untersuchungen zeigten, daß die Zahl der Chromosonen dieser Zellen größer ist als die Zahl der Chromosonen in Lymphocyten oder Myelomzellen aber kleiner als die Summe der Chromosomen beider Fusionspartner.

## Ansprüche

1. Verfahren zur Herstellung von Hybridomzellinien durch Elektrofusion, dadurch gekennzeichnet, daß der für die Durchführung der Elektrofusion notwendige Zellkontakt zwischen den zu fusionierenden Zellen mechanisch hergestellt wird.

2. Verfahren nach Anspruch 1, dadurch gekennzeichnet, daß die Herstellung des Zellkontaktes durch Zentrifugation erfolgt.

3. Verfahren nach Anspruch 1, dadurch gekennzeichnet, daß die Herstellung des Zellkontaktes durch Filtration erfolgt.

4. Verfahren nach Anspruch 1, dadurch gekennzeichnet, daß die Herstellung des Zellkontaktes durch Dialyse erfolgt.

5. Hybridomzellinien, dadurch gekennzeichnet, daß sie mit einem Verfahren gemäß der Ansprüche 1-4 hergestellt werden.

FIG. 1

## Europäisches Patentamt

# EUROPÄISCHER RECHERCHENBERICHT

## EINSCHLÄGIGE DOKUMENTE

EP 86101965.1

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | NATURE, Vol. 310, No. 5980, 30. August 1984 (New York; London) M.M. S. LO et al. "Monoclonal antibody production by receptor-mediated electrically induced cell fusion" Seiten 792-794 <br> * Gesamt * <br> -- | 1,5 | C 12 N 13/00 <br> C 12 N 5/00 <br> C 12 N 15/00 <br> //A 61 K 39/395 |
| A | EP - A2 - 0 128 567 (KERNFORSCHUNGSANLAGE JÜLICH GESELLSCHAFT MIT BESCHRÄNKTER HAFTUNG) <br> * Patentansprüche; Seite 2 * <br> -- | 1,5 | |
| A | EP - A2 - 0 128 565 (KERNFORSCHUNGSANLAGE JÜLICH GESELLSCHAFT MIT BESCHRÄNKTER HAFTUNG) <br> * Patentanspruch 1; Seite 2, letzter Absatz - Seite 3, erster Absatz * <br> ---- | 1,5 | |

RECHERCHIERTE SACHGEBIETE (Int. Cl.4)

C 12 N

A 61 K

Der vorliegende Recherchenbericht wurde für alle Patentansprüche erstellt.

| Recherchenort | Abschlußdatum der Recherche | Prüfer |
|---|---|---|
| WIEN | 05-06-1986 | WOLF |